# EUROPÄISCHE PATENTSCHRIFT

(11) **EP 0 637 321 B1**
(45) Veröffentlichungstag und Bekanntmachung des Hinweises auf die Patenterteilung: **09.07.1997**
(21) Anmeldenummer: 93911463.3
(22) Anmeldetag: 14.04.1993
(51) Int. Cl.: C08F 220/04, A61K 7/48

(54) **VERWENDUNG VON COPOLYMERISATEN AUS CARBONSÄUREN UND LANGKETTIGEN VERBINDUNGEN MIT ISOLIERTEN C-C-MEHRFACHBINDUNGEN ALS VERDICKUNGS- ODER DISPERGIERMITTEL**
USE OF COPOLYMERS OF CARBOXYLIC ACIDS AND LONG-CHAINED COMPOUNDS WITH ISOLATED MULTIPLE C-C BONDS AS THICKENING OR DISPERSING AGENTS
UTILISATION DE COPOLYMERISATS D'ACIDES CARBOXYLIQUES ET DE COMPOSES A CHAINES LONGUES A LIAISONS MULTIPLES C-C ISOLEES COMME EPAISSISSANTS OU COMME DISPERSANTS

(30) Priorität: 23.04.1992 DE 4213283
(43) Veröffentlichungstag der Anmeldung: 08.02.1995
(73) Patentinhaber: BASF Aktiengesellschaft, 67063 Ludwigshafen (DE)
(72) Erfinder: SCHADE, Christian, D-6700 Ludwigshafen (DE); SANNER, Axel, D-6710 Frankenthal (DE); WEKEL, Hans-Ulrich, D-6701 Ellerstadt (DE); FROSCH, Franz, D-6702 Bad Duerkheim (DE); WESTENFELDER, Horst, D-6730 Neustadt (DE)
(86) Internationale Anmeldenummer: EP9300903
(87) Internationale Veröffentlichungsnummer: WO9322357

(56) Entgegenhaltungen:
- EP-A- 0 335 624
- Encyclopedia of Polymer Sience and Engineering, volume 17, Seite 462-468 (1989).

## Beschreibung

Die vorliegende Erfindung betrifft die Verwendung von Copolymerisaten aus Carbonsäuren, langkettigen Verbindungen mit isolierten C-C-Mehrfachbindungen und gegebenenfalls weiteren copolymerisierbaren Monomeren und Vernetzern als Verdikkungs- oder Dispergiermittel z.B. in kosmetischen oder pharmazeutischen Zubereitungen sowie diese Copolymerisate enthaltende kosmetische oder pharmazeutische Zubereitungen. Da ein Teil der Copolymerisate neue Stoffe darstellt, betrifft die Erfindung weiterhin diese neuen Copolymerisate.

Als übliche Verdickungsmittel oder Viskositätsregler werden Copolymerisate aus olefinisch ungesättigten Carbonsäuren wie (Meth)Acrylsäure, Maleinsäure oder Maleinsäureanhydrid und hydrophoben Comonomeren wie (Meth)Acrylsäureestern, α-Olefinen mit 2 bis 12 C-Atomen oder Vinylethern wie Vinylmethylether sowie gegebenenfalls geringen Mengen eines Vernetzers eingesetzt. Derartige Copolymerisate sind beispielsweise in der EP-A 328 725 (1) und der EP-A 435 066 (2) beschrieben.

Aus der US-A 3 755 272 (3) sind Copolymerisate aus unverzweigten α-Olefinen mit 8 bis 30 C-Atomen und (Meth)Acrylsäure bekannt. Diese Copolymerisate eignen sich zur Herstellung von Elektrotauchlackierungen, wasserlöslichen Lacken, Bodenbeschichtungen und Textilbehandlungsmitteln.

Die EP-A 047 009 (4) betrifft Copolymerisate aus 70 bis 93 Gew.-% teil- oder vollneutralisierter Acrylsäure, 7 bis 30 Gew.-% eines α-Olefins mit 6 bis 18 C-Atomen und gegebenenfalls geringen Mengen eines Vernetzungsmittels. Diese Copolymerisate finden Verwendung als wasserabsorbierendes Material in Form von beispielsweise Filmen, Fasern oder Geweben auf dem Sektor der Medizin und Körperpflege. Weiterhin wird das Copolymerisat noch als Flockungsmittel bei der Wasserbehandlung empfohlen.

In der EP-A-0 335 624 sind als Verdicker geeignete oberflächenaktive Polymere beschrieben, die zu 0,5 bis 50 Gew.-% aus ionischen, nichtionischen oder ampholytischen α,β-ungesättigten Monomeren bestehen, welche mindestens eine aliphatische oder araliphatische lipophile Gruppe mit C₈-C₃₀-C-Atomen tragen, und zu 40-99 Gew.-% aus weiteren nicht lipophilen α,β-ungesättigten Monomeren. Bei den lipophilen Monomeren handelt es sich überwiegend um ionische Monomere, daneben auch um nichtionische (Meth)acrylester oder um Allyletherverbindungen.

Die aus dem Stand der Technik bekannten Verdickungsmittel oder Viskositätsregler weisen eine Reihe von Nachteilen auf. So sind diese Mittel oft nicht ausreichend hydrolysestabil und neigen unter den Anwendungsbedingungen zu Zersetzungen. Einige dieser Mittel weisen ein nicht unerhebliches toxisches Potential auf, was bei einer kosmetischen Verwendung von besonderer Bedeutung ist. Auch ist häufig die Stabilität der mit diesen Mitteln hergestellten kosmetischen Zubereitungen, insbesondere von Emulsionen, noch nicht optimal, vor allem die Stabilität gegenüber Elektrolyten ist verbesserungsbedürftig.

Aufgabe der vorliegenden Erfindung war es daher, neue Verdickungs- und Dispergiermittel für kosmetische Zubereitungen bereitzustellen, welche die geschilderten Nachteile des Standes der Technik nicht mehr aufweisen.

Demgemäß wurde die Verwendung von Copolymerisaten, welche erhältlich sind durch radikalisch initiierte Polymerisation von
A) 75 bis 99,45 Gew.-% einer olefinisch ungesättigten C₃- bis C₅-Monocarbonsäure, einer olefinisch ungesättigten C₄- bis C₈-Dicarbonsäure oder ihres Anhydrids oder einer Mischung solcher Carbonsäuren oder Carbonsäureanhydride mit
B) 0,5 bis 24,95 Gew.-% einer oder mehrerer langkettiger Verbindungen mit isolierten C-C-Mehrfachbindungen aus der Gruppe
   (1) ein- oder mehrfach ungesättigter C₈- bis C₃₀-Monocarbonsäuren, welche zusätzliche Hydroxylgruppen tragen können, sowie deren Alkalimetall- und Erdalkalimetallsalzen, Alkylestern, Amiden, Sorbitanestern, Glycerinestern oder Polyglycerinestern,
   (2) ein- oder mehrfach ungesättigter aliphatischer C₈- bis C₃₀-Amine,
   (3) ein- oder mehrfach ungesättigte C₈- bis C₃₀-Alkohole sowie deren Estern mit gesättigten C₁- bis C₄-Monocarbonsäuren,
   (4) end- und mittelständiger C₁₆- bis C₃₀-Alkene,
C) 0 bis 24,45 Gew.-% weiterer copolymerisierbarer Monomerer und
D) 0,05 bis 5 Gew.-% einer oder mehrerer Verbindungen mit mindestens zwei olefinisch ungesättigten Gruppen im Molekül als Vernetzer,
als Verdickungs- oder Dispergiermittel gefunden.

In einer bevorzugten Ausführungsform ist das erfindungsgemäß verwendete Copolymerisat aufgebaut aus
A) 94 bis 98,9 Gew.-% der Carbonsäure-Komponente A,
B) 1 bis 5,9 Gew.-% der langkettigen Verbindungen mit isolierten C-C-Mehrfachbindungen B,
C) 0 bis 4,9 Gew.-% weiterer copolymerisierbarer Monomerer und
D) 0,1 bis 2,5 Gew.-% der Vernetzer-Komponente D.

Als Komponente A eignen sich vor allem Acrylsäure, Methacrylsäure oder Maleinsäureanhydrid, daneben aber auch Crotonsäure, 2-Pentensäure, Maleinsäure, Fumarsäure oder Itaconsäure.

Unter langkettigen Verbindungen mit isolierten C-C-Mehrfachbindungen B sind solche mit isolierten acetylenischen Dreifachbindungen und insbesondere solche mit isolierten olefinischen Doppelbindungen zu verstehen. Unter "isoliert" ist zu verstehen, daß beim Vorliegen mehrerer solcher C-C-Mehrfachbindungen diese nicht konjugiert sind und auch nicht mit funktionellen Gruppen mit π-Elektronensystemen, z.B. Carboxyl- oder Carbonylgruppen, in Wechselwirkung treten.

Als langkettige Carbonsäuren (1) für die Komponente B kommen insbesondere natürlich vorkommende ungesättigte Fettsäuren, beispielsweise Ölsäure, Nervonsäure, a-Hydroxynervonsäure, Elaidinsäure, Erucasäure, Stearolsäure, Palmitoleinsäure, Vaccensäure, Linolsäure, Linolensäure, Petroselinsäure, Arachidonsäure oder Ricinolsäure, in Betracht. Bevorzugt werden hiervon C₁₄- bis C₂₄-Monocarbonsäuren mit 1 bis 4 C-C-Mehrfachbindungen, insbesondere olefinischen Doppelbindungen. Die Carbonsäuren (1) können in der Säureform oder als Alkalimetallsalze, insbesondere Natrium- oder Kaliumsalze, oder als Erdalkalimetallsalze, insbesondere als Calciumsalze, eingesetzt werden.

Als Ester langkettiger Carbonsäuren (1) dienen die C₁- bis C₄-Alkylester wie die Methyl-, Ethyl-, n-Propyl-, n-Butyl- oder tert.-Butylester, z.B. Ölsäuremethylester, die Ester langkettiger C₁₄- bis C₂₄-Alkohole mit 1 bis 4 C-C Mehrfachbindungen, insbesondere olefinischen Doppelbindungen, wie Ölsäureoleylester, Sorbitanester wie Sorbitanmono- oder Sorbitansesquioleat sowie die Glycerin- und Polyglycerinester, z.B. Mono-, Di- und Triglyceride wie Glycerintrioleat, also auch natürlich vorkommende Fette (Lipide).

Geeignet sind weiterhin Amide aus den beschriebenen Carbonsäuren und zahlreichen Aminen, beispielsweise Ölsäurediethanolamid, -isopropanolamid oder -dibutylamid.

Als langkettige Amine (2) für die Komponente B eignen sich insbesondere aliphatische primäre C₁₄- bis C₂₄-Amine mit 1 bis 4 C-C-Mehrfachbindungen, insbesondere olefinischen Doppelbindungen, z.B. Oleylamin.

Als langkettige Alkohole (3) für die Komponente B dienen vor allem primäre C₁₄- bis C₂₄-Alkohole mit 1 bis 4 C-C-Mehrfachbindungen, insbesondere olefinischen Doppelbindungen, sowie Ester hieraus mit gesättigten C₁- bis C₄-Monocarbonsäuren wie Ameisensäure, Essigsäure, Propionsäure oder Buttersaure. Beispiele für (3) sind Oleylalkohol und Oleylacetat.

Ebenfalls gut geeignet als Komponente B sind Öle natürlichen Ursprungs, insbesondere Öle pflanzlichen Ursprungs, sowie natürliche Wachse und tierische Talge, Fette und Schmalze, welche die genannten Verbindungen (1) bis (3) enthalten. Geeignete Beispiele hierfür sind Kokos-, Palmkern-, Palm-, Erdnuß-, Soja-, Rüb-, Sonnenblumen-, Igoba-, Oliven-, Sesam-, Baumwollsamen-, Lein-, Distel-, Mais-, Ricinus- oder Fischöl.

Als langkettige end- und mittelständige Alkene (4) eignen sich insbesondere endständige C₁₆- bis C₂₄-Alkene (α-Olefine). Beispiele für (5) sind 1-Hexadecen, 1-Octadecen, 1-Eicosaen, 1-Tetracosen sowie C₂₀/C₂₄- und C₂₀/C₃₀-Gemische oder -Schnitte der entsprechenden Olefin-Fraktionen.

Als weitere copolymerisierbare Monomere C zur geringfügigen Modifizierung der erfindungsgemäß verwendeten Copolymerisate eignen sich beispielsweise N-Vinylpyrrolidon, N-Vinylcaprolactam, C₁- bis C₁₈-Alkyl(meth)acrylate, z.B. Methyl(meth)acrylat, Ethyl (meth) acrylat oder Stearyl(meth)acrylat, (Meth)Acrylamid oder N-(C₁- bis C₁₈-Alkyl) (meth)acrylamide, z.B. N,N-Dimethyl(meth)acrylamid, N-tert.-Butyl(meth)acrylamid oder N-tert.-Octyl(meth)acrylamid, Vinylester von C₁-C₁₈-Carbonsäuren, z.B. Vinylacetat, Vinylpropionat, Versaticsäurevinylester, Hydroxyalkylenmono(meth)acrylester mit zwei bis sechs Kohlenstoffatomen in der Alkylenkette oder -ethylalkoholen mit 1 bis 25 Ethylenoxideinheiten im Molekül, z.B. Ethyldiglycolacrylat.

Als Vernetzer-Komponente D dienen olefinisch mehrfach ungesättigte Verbindungen wie insbesondere Divinylbenzol, Divinylethylenharnstoff, Diallylweinsäurediamid, Methylenbisacrylamid, (Meth)Acrylester von mehrfunktionellen Alkoholen wie Trimethylolpropan, Pentaerythrit, Alkylenglykolen mit 2 bis 6 C-Atomen in der Alkylengruppe, Polyethylenglykolen oder Polypropylenglykolen, Allylester der (Meth)Acrylsäure, Oleyl(meth)acrylat, Oleyl(meth)acrylamid, Trivinylcyclohexan, Triallyltriazintrion und Allylether von Trimethylolpropan, Pentaerythrit und Saccharose mit mindestens zwei Allylethereinheiten pro Molekül. Besonders bevorzugt werden Pentaerythrittriallylether, Pentaallylsaccharose, Methacrylsäureallylester, Trimethylolpropandiallylether und Methylenbisacrylamid.

Zur Herstellung der erfindungsgemäß verwendeten Copolymerisate können die Monomere A bis D im Prinzip nach allen bekannten Verfahren polymerisiert werden. Eine besonders gut geeignete Herstellungsmethode ist die Fällungspolymerisation, bei der die Monomeren, nicht aber das Polymere im eingesetzten Lösungsmittel-System löslich sind. Geeignete Lösungsmittel sind aromatische wie Toluol oder Xylol, halogenierte wie 1,1,1-Trichlorethan oder Methylenchlorid sowie insbesondere semipolare Solventien wie Ketone mit 3 bis 6 C-Atomen und C₂- bis C₆-Alkylester der Ameisen- und Essigsäure, weiterhin unpolare Kohlenwasserstoffe, z.B. Cyclohexan oder Petrolether sowie Mischungen dieser Lösungsmittel. Besonders gut geeignet sind auch aromatische und aliphatische Kohlenwasserstoffe mit 5 bis 12 Kohlenstoffatomen im Molekül.

Die Polymerisation wird in Gegenwart einer Radikale bildenden Verbindung wie organischen Azo- oder Peroxoverbindungen durchgeführt. Geeignete Initiatoren sind z.B. Diacylperoxide wie Dilauroyl-, Didecanoyl- und Dioctanoylperoxid oder Perester wie tert.-Butylperoctanoat, tert.-Butylperpivalat, tert.-Amylperpivalat oder tert.-Butylperneodecanoat sowie Azoverbindungen wie Dimethyl-2,2'-azobis(isobutyrat), 2,2'-Azobis(isobutyronitril), 2,2'-Azobis(2-methylbutyronitril) oder 2,2'-Azobis(2,4-dimethylvaleronitril).

Der Polymerisationsmischung können in geringer Menge Wasser, Alkohole, Schutzkolloide, Emulgatoren oder auch größere Mengen einer Base, z.B. Kaliumcarbonat, zugesetzt werden. Das Molekulargewicht der Polymeren kann gewünschtenfalls durch Zugabe von Reglern zur Polymerisationsmischung erniedrigt werden.

Der Polymerisationsprozeß wird bevorzugt so gesteuert, daß das Polymer in Form eines feinteiligen Pulvers anfällt, das gegebenenfalls einem geeigneten Trenn-, Trocknungs- oder Mahlverfahren unterworfen wird.

Ziel der erfindungsgemäßen Verwendung der beschriebenen Copolymerisate ist vor allem der Einsatz dieser Substanzen als Verdicker, Gelbildner und als Emulgatoren für technische, pharmazeutische und insbesondere kosmetische Anwendungen, beispielsweise in Cremes, Lotionen oder Gelen. Die beschriebenen Copolymerisate eignen sich gut zum Verdicken wäßriger Systeme unter Ausbildung verdickter Gele, nachdem das dispergierte Polymer durch Zugabe einer Base wie z.B. Triethanolamin, NaOH, KOH, 2-Amino-2-methyl-1-propanol, 2-Amino-2-methyl-1,3-propandiol, Diisopropanolamin oder Tetrahydroxypropylethylendiamin ausreichend neutralisiert worden ist. Auf ähnliche Weise lassen sich die Polymere zur Herstellung sehr stabiler Emulsionen aus einer Wasser- und einer Ölphase, insbesondere von Öl-in-Wasser-Emulsionen, verwenden. Gegenüber herkömmlichen Emulgatoren genügen im allgemeinen geringere Einsatzmengen des Polymeren, um dauerhaft stabile Emulsionen zu erhalten.

Gegenstand der vorliegenden Erfindung sind weiterhin kosmetische Zubereitungen, welche die erfindungsgemäß verwendeten Copolymerisate als Verdickungs- oder Dispergiermittel in den hierfür üblichen Mengen, also etwa 0,05 bis 2 Gew.-%, enthalten.

Ein Teil der erfindungsgemäß verwendeten Copolymerisate stellt neue Stoffe dar, deshalb betrifft die Erfindung weiterhin Copolymerisate, welche erhältlich sind durch radikalisch initiierte Polymerisation von
A) 50 bis 99,9 Gew.-% einer olefinisch ungesättigten C₃- bis C₅-Monocarbonsäure, einer olefinisch ungesättigten C₄- bis C₈-Dicarbonsäure oder ihres Anhydrids oder einer Mischung solcher Carbonsäuren oder Carbonsäureanhydride mit
B) 0,1 bis 50 Gew.-% einer oder mehrerer langkettiger Verbindungen mit isolierten C-C-Mehrfachbindungen aus der Gruppe
   (1) ein- oder mehrfach ungesättigte C₈- bis C₃₀-Monocarbonsäure, welche zusätzliche Hydroxylgruppen tragen können, sowie deren Alkalimetall- und Erdalkalimetallsalzen, Alkylestern, Amiden, Sorbitanestern, Glycerinestern oder Polyglycerinestern,
   (2) ein- oder mehrfach ungesättigter aliphatischer C₈- bis C₃₀-Amine,
   (3) ein- und mehrfach ungesättigter C₈- bis C₃₀-Alkohole sowie Ester hieraus mit gesättigten C₁- bis C₄-Monocarbonsäuren und
   (4) der (C₈- bis C₃₀-Alkyl)vinylether, welche bis zu 25 Alkylenoxid-Einheiten eingebaut enthalten können,
C) 0 bis 49,9 Gew.-% weiterer copolymerisierbarer Monomerer und
D) 0 bis 10 Gew.-% einer oder mehrerer Verbindungen mit mindestens zwei olefinisch ungesättigten Gruppen im Molekül als Vernetzer.

Die erfindungsgemäß verwendeten Copolymerisate weisen eine Reihe von Vorteilen auf. Aufgrund der chemischen Natur der langkettigen Comonomere B können die hydrophoben Anteile des Polymers nicht hydrolytisch entfernt werden; die besondere Verdicker- und Dispergierwirkung bleibt deshalb auch unter stark hydrolytischen Bedingungen erhalten. Die Comonomeren B haben außerdem ein deutlich geringeres toxisches Potential als beispielsweise die aus dem Stand der Technik als Comonomere bekannten Acrylatverbindungen.

Die Synthese der erfindungsgemäß verwendeten Copolymerisate gelingt überraschenderweise gut, obwohl einerseits Alkylvinylether in Gegenwart sauer reagierender Verbindungen bekanntermaßen zur kationischen Autopolymerisation oder Hydrolyse neigen und andererseits Verbindungen mit isolierten olefinischen Doppelbindungen, insbesondere Verbindungen mit mittel ständigen olefinischen Doppelbindungen, unter radikalischen Bedingungen als schlecht polymerisierbar gelten, da sie unter üblichen Bedingungen oft nur ungenügend homopolymerisierbar sind oder selbst als regelnde Komponente bei der Polymerisation anderer Monomerer bekannt sind.

### Beispiele

Soweit nichts anderes angegeben ist, beziehen sich die Prozentangaben auf das Gewicht.

### Beispiel 1

In einem 3 1-Planschliffkolben wurden 1400 ml 1,1,1-Trichlorethan, 250 g Acrylsäure, 1,5 g Pentaerythrittriallylether und 10 g 1-Octadecen verrührt und 30 min mit Stickstoff gespült. Im Stickstoff-Strom wurde auf 80°C unter Rühren erwärmt und nach Erreichen dieser Temperatur während 3 h ein Zulauf aus 100 ml 1,1,1-Trichlorethan und 0,4 g Dilauroylperoxid zugegeben. Nach weiteren 3 h wurde abgekühlt, das ausgefallene Produkt abfiltriert, mit 500 ml 1,1,1-Trichlorethan gewaschen und im Vakuum bei 60°C getrocknet.

Zur Bestimmung der Gelviskosität wurde in einem Becherglas 1,0 g des Polymeren in 190 ml Wasser dispergiert. Unter Rühren wurden 10 ml einer 10 %igen Triethanolamin-Lösung zugegeben. Die Viskosität des erhaltenen Gels wurde mit einem Handviskosimeter (Hake VT-02) zu 8,0 Pa.s bestimmt. Durch Ausstreichen auf eine Glasplatte konnte man erkennen, daß das Gel glatt und annähernd stippenfrei war.

Zur Kontrolle der Emulgierfähigkeit wurden 0,4 g des Polymeren in ein Becherglas eingewogen und in 30 ml Paraffinöl dispergiert. Danach wurden 100 ml Wasser und anschließend 4 ml einer 10 %igen Triethanolamin-Lösung unter intensivem Rühren zugegeben. Die Emulsion wurde mit einem Dispergieraggregat bei 8000 U/min für wenige s homogenisiert. Die Viskosität wurde wie oben zu 17,6 Pa.s bestimmt. Die Struktur der Emulsion wurde nach 1 h durch Ausstreichen auf eine Glasplatte begutachtet. Zur Bestimmung der Langzeitstabilität wurde die Emulsion in einen 100 ml-Standzylinder gefüllt und nach 14 d bewertet. Die Emulsion zeigte zu diesem Zeitpunkt keine Tendenz, sich zu trennen.

### Beispiele 2 bis 19

In Analogie hierzu wurden die Beispiele 2 bis 19 durchgeführt. Tabelle 1 zeigt die Ergebnisse.

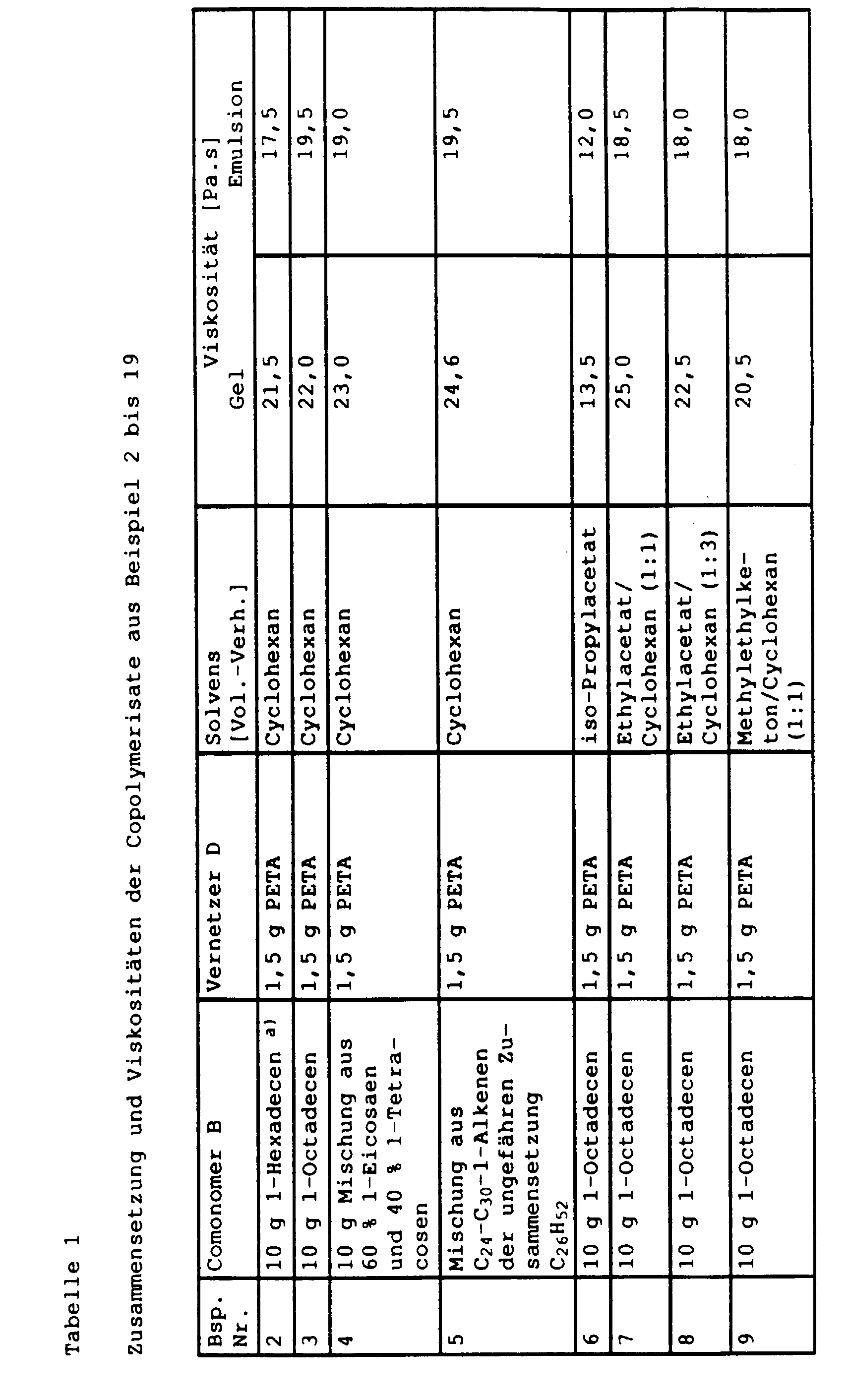

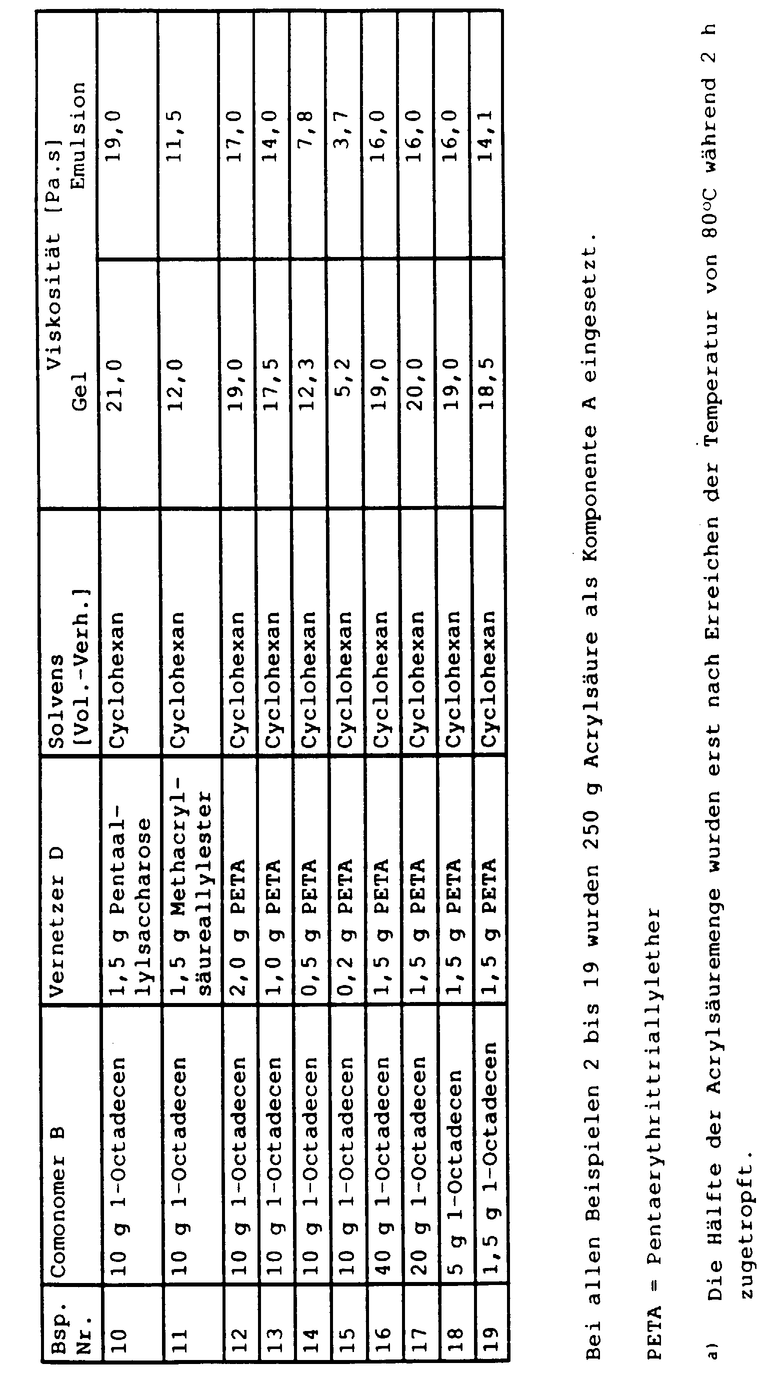

### Beispiel 20

In einem 3 1-Planschliffkolben wurden 1400 ml 1,1,1-Trichlorethan, 250 g Acrylsäure, 1,5 g Pentaerythrittriallylether und 10 g Ölsäure verrührt und 30 min mit Stickstoff gespült. Im Stickstoff-Strom wurde auf 80°C unter Rühren erwärmt und nach Erreichen dieser Temperatur während 3 h ein Zulauf aus 100 ml 1,1,1-Trichlorethan und 0,4 g Dilauroylperoxid zugegeben. Nach weiteren 3 h wurde abgekühlt, das ausgefallene Produkt filtriert, mit 500 ml 1,1,1-Trichlorethan gewaschen und im Vakuum bei 60°C getrocknet.

Zur Bestimmung der Gelviskosität wurde in einem Becherglas 1,0 g des Polymeren in 190 ml Wasser dispergiert. Unter Rühren wurden 10 ml einer 10 %igen Triethanolamin-Lösung zugegeben. Die Viskosität des erhaltenen Gels wurde mit einem Handviskosimeter (Hake VT-02) zu 8,0 Pa.s bestimmt. Durch Ausstreichen auf eine Glasplatte konnte man erkennen, daß das Gel glatt und annähernd stippenfrei war.

Zur Kontrolle der Emulgierfähigkeit wurden 0,4 g des Polymeren in ein Becherglas eingewogen und in 30 ml Paraffinöl dispergiert. Danach wurden 100 ml Wasser und anschließend 4 ml einer 10 %igen Triethanolamin-Lösung unter intensivem Rühren zugegeben. Die Emulsion wurde mit einem Dispergieraggregat bei 8000 U/min für wenige s homogenisiert. Die Viskosität wurde wie oben zu 7,6 Pa.s bestimmt. Die Struktur der Emulsion wurde nach 1 h durch Ausstreichen auf eine Glasplatte begutachtet. Zur Bestimmung der Langzeitstabilität wurde die Emulsion in einen 100 ml-Standzylinder gefüllt und nach 14 d bewertet. Die Emulsion zeigte zu diesem Zeitpunkt keine Tendenz, sich zu trennen.

### Beispiele 21 bis 61

In Analogie hierzu wurden die Beispiele 21 bis 61 durchgeführt. Tabelle 2 zeigt die Ergebnisse.

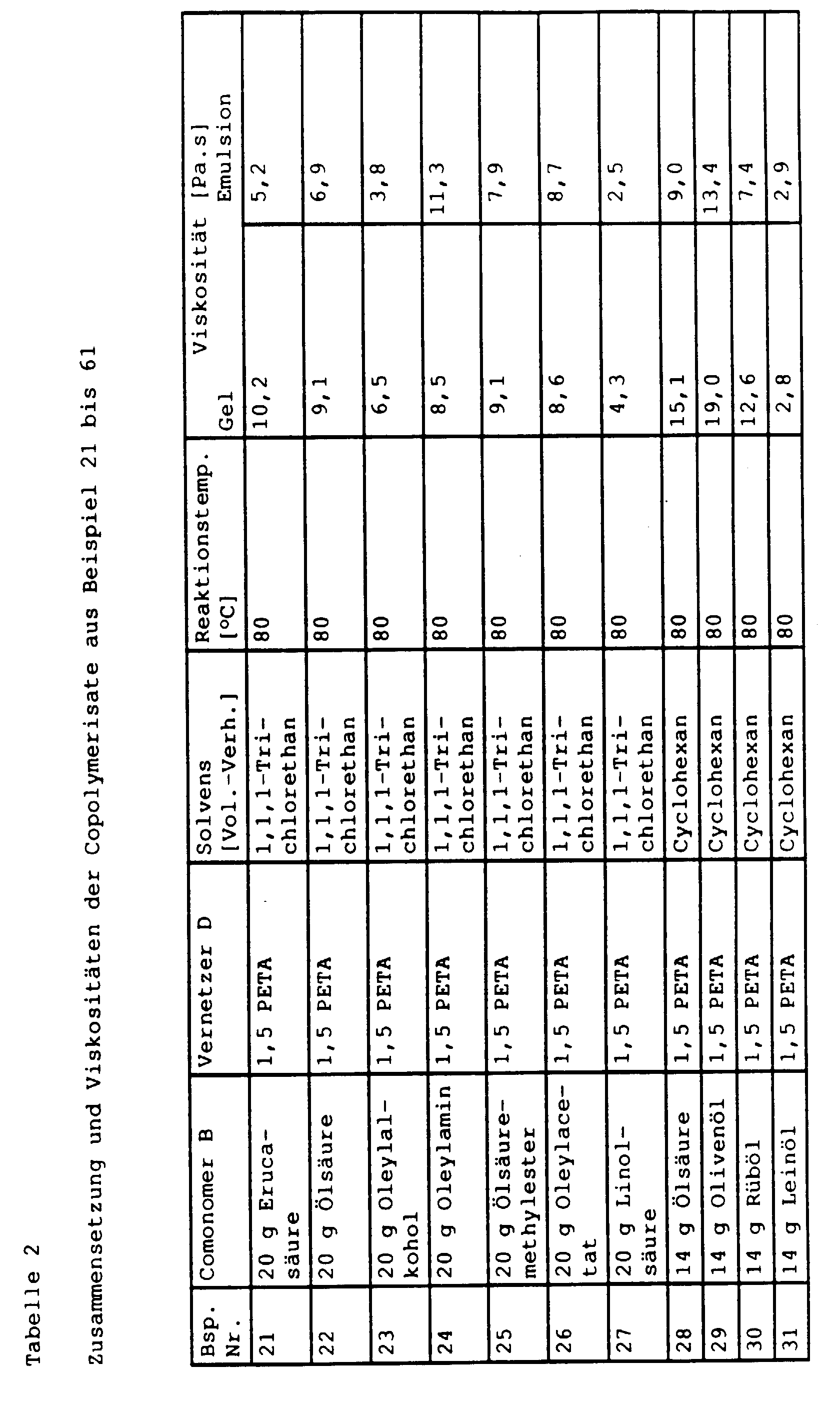

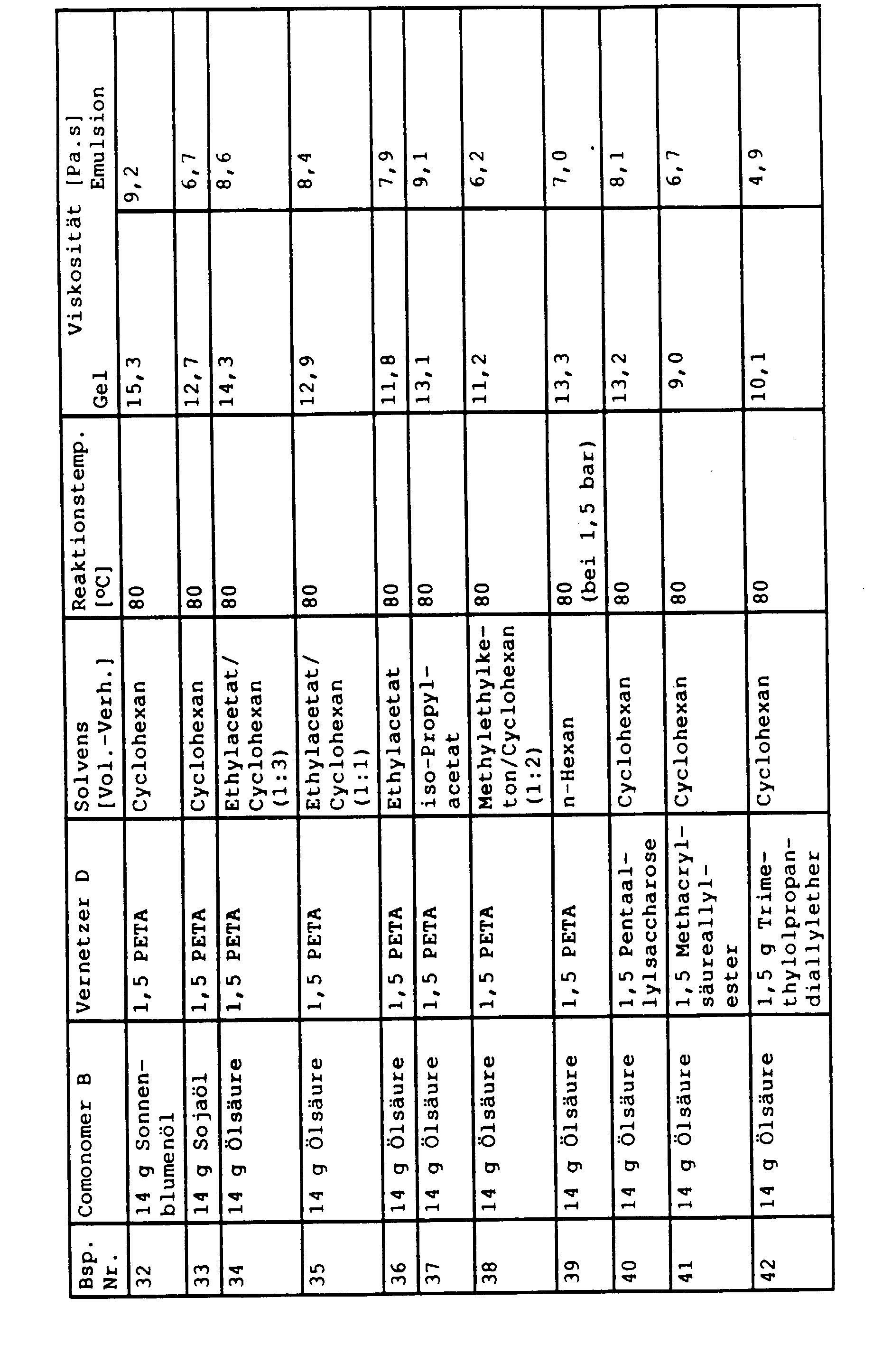

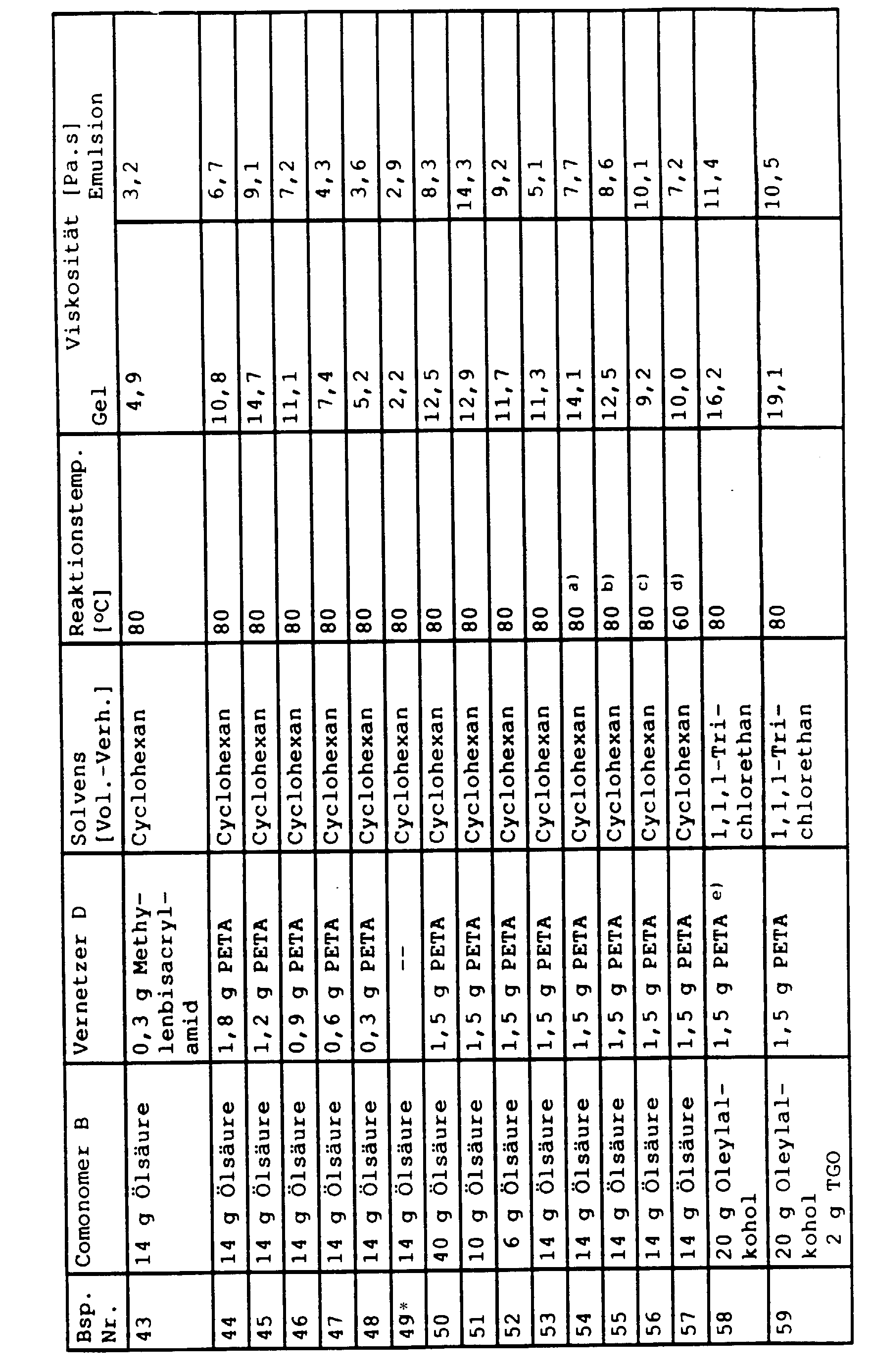

### Beispiel 62

In einem 3 1 Planschliffkolben wurden 1400 ml 1,1,1-Trichlorethan, 250 g Acrylsäure, 1,5 g Pentaerythrittriallylether und 10 g Octadecylvinylether verrührt und 30 min mit Stickstoff gespült. Im Stickstoff-Strom wurde auf 80°C unter Rühren erwärmt und nach Erreichen dieser Temperatur während 3 h ein Zulauf aus 100 ml 1,1,1-Trichlorethan und 0,4 g Dilauroylperoxid zugegeben. Nach weiteren 3 h wurde abgekühlt, das ausgefallene Produkt abfiltriert, mit 500 ml 1,1,1-Trichlorethan gewaschen und im Vakuum bei 60°C getrocknet.

Zur Bestimmung der Gelviskosität wurde 1,0 g des Polymeren in einem Becherglas in 190 ml Wasser dispergiert. Unter Rühren wurden 10 ml einer 10 %igen Triethanolamin-Lösung zugegeben. Die Viskosität des erhaltenen Gels wurde mit einem Handviskosimeter (Hake VT-02) zu 10,0 Pa.s bestimmt. Durch Ausstreichen auf eine Glasplatte konnte man erkennen, daß das Gel glatt und annähernd stippenfrei war.

Zur Kontrolle der Emulgierfähigkeit wurden 0,4 g des Polymeren in ein Becherglas eingewogen und in 30 ml Paraffinöl dispergiert. Danach wurden 100 ml Wasser und anschließend 4 ml einer 10 %igen Triethanolamin-Lösung unter intensivem Rühren zugegeben. Die Emulsion wurde mit einem Dispergieraggregat bei 8000 U/min für wenige s homogenisiert. Die Viskosität wurde wie oben zu 8,0 Pa.s bestimmt. Die Struktur der Emulsion wurde nach 1 h durch Ausstreichen auf eine Glasplatte begutachtet. Zur Bestimmung der Langzeitstabilität wurde die Emulsion in einen 100 ml-Standzylinder gefüllt und nach 14 d bewertet. Die Emulsion zeigte zu diesem Zeitpunkt keine Tendenz, sich zu trennen.

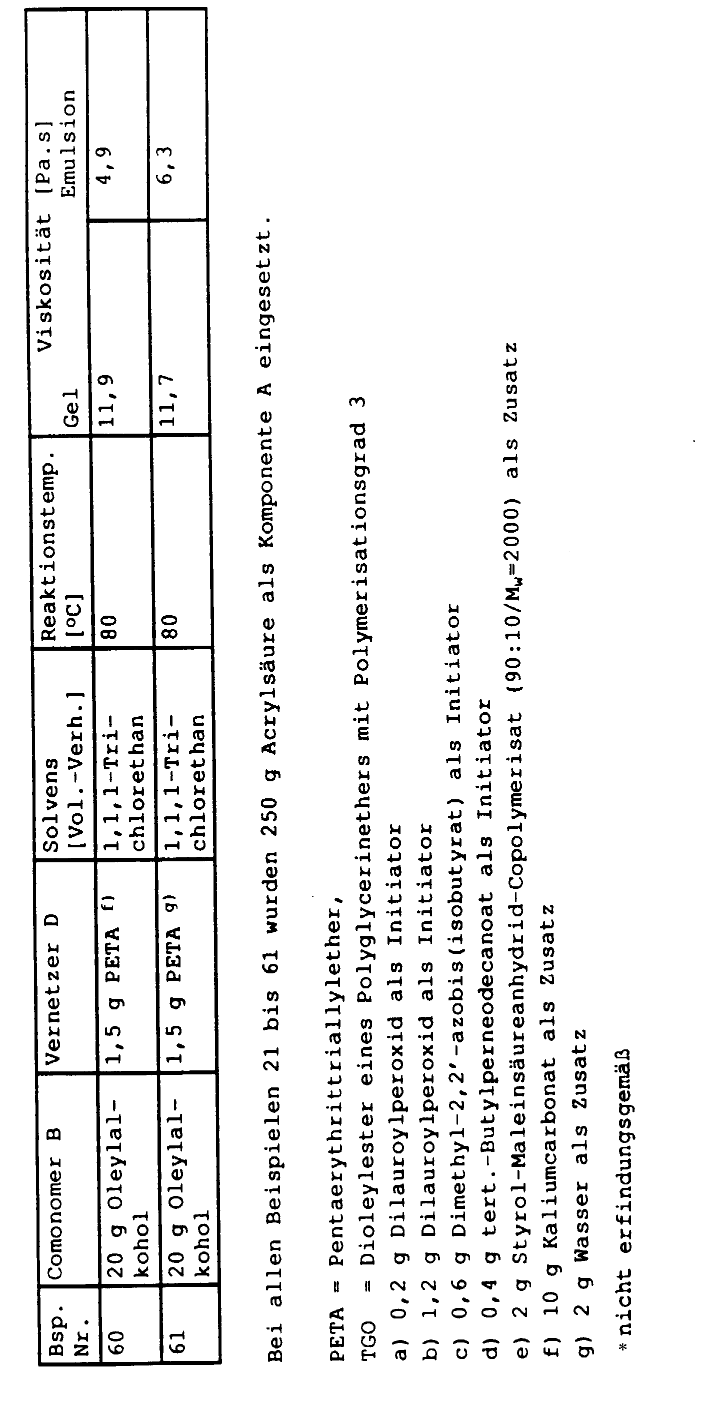

## Patentansprüche

1. Verwendung von Copolymerisaten, erhältlich durch radikalisch initiierte Polymerisation von
A) 75 bis 99,45 Gew.-% einer olefinisch ungesättigten C₃- bis C₅-Monocarbonsäure, einer olefinisch ungesättigten C₄- bis C₈-Dicarbonsäure oder ihres Anhydrids oder einer mischung solcher Carbonsäuren oder Carbonsäureanhydride mit
B) 0,5 bis 24,95 Gew.-% einer oder mehrerer langkettiger Verbindungen mit isolierten C-C-Mehrfachbindungen aus der Gruppe
(1) ein- oder mehrfach ungesättigter C₈- bis C₃₀-Monocarbonsäuren, welche zusätzliche Hydroxylgruppen tragen können, sowie deren Alkalimetall- und Erdalkalimetallsalzen, Alkylestern, Amiden, Sorbitanestern, Glycerinestern oder Polyglycerinestern,
(2) ein- oder mehrfach ungesättigter aliphatischer C₈- bis C₃₀-Amine,
(3) ein- oder mehrfach ungesättigter C₈- bis C₃₀-Alkohole sowie deren Estern mit gesättigten C₁- bis C₄-Monocarbonsäuren,
(4) end- oder mittelständiger C₁₆- bis C₃₀-Alkene,
C) 0 bis 24,45 Gew.-% weiterer copolymerisierbarer Monomerer und
D) 0,05 bis 5 Gew.-% einer oder mehrerer Verbindungen mit mindestens zwei olefinisch ungesättigten Gruppen im Molekül als Vernetzer,
als Verdickungs- oder Dispergiermittel.

2. Verwendung von Copolymerisaten nach Anspruch 1, bei deren Herstellung als Komponente A Acrylsäure, Methacrylsäure oder Maleinsäureanhydrid verwendet wurden.

3. Verwendung von Copolymerisaten nach den Ansprüchen 1 bis 2, bei deren Herstellung als Komponente B eine oder mehrere langkettige Verbindungen mit isolierten olefinischen Doppelbindungen aus der Gruppe
(1) ein bis vierfach ungesättigter C₁₄- bis C₂₄-Monocarbonsäuren sowie deren Alkalimetall- und Erdalkalimetallsalzen, C₁- bis C₄-Alkylestern, Glycerinestern oder Polyglycerinestern,
(2) ein- bis vierfach ungesättigte aliphatischer primärer C₁₄- bis C₂₄-Amine,
(3) ein- bis vierfach ungesättigter primärer C₁₄- bis C₂₄-Alkohole sowie deren Estern mit gesättigten C₁- bis C₄-Monocarbonsäuren,
(4) endständiger C₁₆- bis C₂₄-Alkene
eingesetzt wurden.

4. Verwendung von Copolymerisaten nach den Ansprüchen 1 bis 3, bei deren Herstellung als Komponente D Allylether von Pentaerythrit, Trimethylolpropan oder Saccharose mit mindestens zwei Allylethereinheiten im Molekül sowie Methacrylsäureallylester, Oleyl(meth)acrylat oder Methylenbisacrylamid verwendet wurden.

5. Verwendung von Copolymerisaten gemäß Anspruch 1 als Verdickungs- oder Dispergiermittel in kosmetischen Zubereitungen.

6. Verwendung von Copolymerisaten gemäß Anspruch 1 als Verdickungs- oder Dispergiermittel in pharmazeutischen Zubereitungen.

7. Copolymerisate, erhältlich durch radikalisch initiierte Polymerisation von
(A) 75 bis 99,45 Gew.-% einer olefinisch ungesättigten C₃- bis C₅-Monocarbonsäure, einer olefinisch ungesättigten C₄- bis C₈-Dicarbonsäure oder ihres Anhydrids oder einer Mischung solcher Carbonsäuren oder Carbonsäureanhydride mit
(B) 0,5 bis 24,95 Gew.-% einer oder mehrerer langkettiger Verbindungen mit isolierten C-C-Mehrfachbindungen aus der Gruppe
(1) ein- oder mehrfach ungesättigter C₈- bis C₃₀-Monocarbonsäuren, welche zusätzliche Hydroxylgruppen tragen können, sowie deren Alkalimetall- und Erdalkalimetallsalzen, C₁- bis C₄-Alkylestern, Glycerinestern oder Polyglycerinestern,
(2) ein- oder mehrfach ungesättigter aliphatischer C₈- bis C₃₀-Amine und
(3) ein- und mehrfach ungesättigter C₈- bis C₃₀-Alkohole sowie deren Estern mit gesättigten C₁- bis C₄-Monocarbonsäuren,
(C) 0 bis 24,45 Gew.-% weiterer copolymerisierbarer Monomerer und
(D) 0,05 bis 5 Gew.- einer oder mehrerer Verbindungen mit mindestens zwei olefinisch ungesättigten Gruppen im Molekül als Vernetzer.

8. Kosmetische oder pharmazeutische Zubereitungen, enthaltend Copolymerisate gemäß den Ansprüchen 1 bis 4 als Verdickungs- und Dispergiermittel in den hierfür üblichen Mengen.

## Claims

1. The use of copolymers obtainable by free-radical polymerization of
A) 75 - 99.45% by weight of an olefinically unsaturated C₃-C₅-monocarboxymic acid, of an olefinically unsaturated C₄-C₈-dicarboxylic acid or of its anhydride or a mixture of such carboxylic acids or anhydrides with
B) 0.5 - 24.95% by weight of one or more long-chain compounds with isolated C-C multiple bonds from the group comprising
(1) mono- or polyunsaturated C₈-C₃₀-monocarboxylic acids which may have additional hydroxyl groups, as well as their alkali metal and alkaline earth metal salts, alkyl esters, amides, sorbitan esters, glycerol esters or polyglycerol esters,
(2) mono- or polyunsaturated aliphatic C₈-C₃₀-amines,
(3) mono- or polyunsaturated C₈-C₃₀-alcohols as well as their esters with saturated C₁-C₄-monocarboxylic acids,
(4) terminal or internal C₁₆-C₃₀-alkenes,
C) 0 - 24.45% by weight of other copolymerizable monomers and
D) 0.05 - 5% by weight of one or more compounds with at least two olefinically unsaturated groups in the molecule as crosslinkers,
as thickeners or dispersants.

2. The use of copolymers as claimed in claim 1, prepared using acrylic acid, methacrylic acid or maleic anhydride as component A.

3. The use of copolymers as claimed in claim 1 or 2, prepared using as component B one or more long-chain compounds with isolated olefinic double bonds from the group comprising
(1) mono- to tetraunsaturated C₁₄-C₂₄-monocarboxylic acids as well as their alkali metal and alkaline earth metal salts, C₁-C₄-alkyl esters, glycerol esters or polyglycerol esters,
(2) mono- to tetraunsaturated aliphatic primary C₁₄-C₂₄-amines,
(3) mono- to tetraunsaturated primary C₁₄-C₂₄-alcohols as well as their esters with saturated C₁-C₄-monocarboxylic acids,
(4) terminal C₁₆-C₂₄-alkenes.

4. The use of copolymers as claimed in claims 1 to 3, prepared using as component D allyl ethers of pentaerythritol, trimethylolpropane or sucrose with at least two allyl ether units in the molecule as well as allyl methacrylate, oleyl (meth)acrylate or methylenebisacrylamide.

5. The use of copolymers as claimed in claim 1 as thickeners or dispersants in cosmetic preparations.

6. The use of copolymers as claimed in claim 1 as thickeners or dispersants in pharmaceutical preparations.

7. A copolymer obtainable by free-radical polymerization of
(A) 75 - 99.45% by weight of an olefinically unsaturated C₃-C₅-monocarboxylic acid, of an olefinically unsaturated C₄-C₈-dicarboxylic acid or of its anhydride or a mixture of such carboxylic acids or anhydrides with
(B) 0.5 - 24.95% by weight of one or more long-chain compounds with isolated C-C multiple bonds from the group comprising
(1) mono- or polyunsaturated C₈-C₃₀-monocarboxylic acids which may have additional hydroxyl groups, as well as their alkali metal and alkaline earth metal salts, C₁-C₄-alkyl esters, glycerol esters or polyglycerol esters,
(2) mono- or polyunsaturated aliphatic C₈-C₃₀-amines and
(3) mono- and polyunsaturated C₈-C₃₀-alcohols as well as their esters with saturated C₁-C₄-monocarboxylic acids,
(C) 0 - 24.45% by weight of other copolymerizable monomers and
(D) 0.05 - 5% by weight of one or more compounds with at least two olefinically unsaturated groups in the molecule as crosslinkers.

8. A cosmetic or pharmaceutical preparation containing copolymers as claimed in claims 1 to 4 as thickeners and dispersants in the amounts customary for this purpose.

## Revendications

1. Utilisation de copolymères qui peuvent être obtenus par la polymérisation amorcée par voie radicalaire de
A) 75 à 99,45% en poids d'un acide monocarboxylique en C₃ à C₅, oléfiniquement insaturé, d'un acide dicarboxylique en C₄ à C₈, oléfiniquement insaturé, ou de son anhydride, ou d'un mélange de tels acides carboxyliques ou anhydrides d'acides carboxyliques avec
B) 0,5 à 24,95% en poids d'un ou plusieurs composés à longues chaînes avec de multiples liaisons C-C, appartenant au groupe formé par
(1) des acides monocarboxyliques en C₈ à C₃₀, mono- ou polyinsaturés, qui peuvent porter des radicaux hydroxyle complémentaires, ainsi que leurs sels de métaux alcalins et de métaux alcalino-terreux, leurs esters alkyliques, leurs amides, leurs esters avec le sorbitan, leurs esters avec la glycérine, ou leurs esters avec des polyglycérines,
(2) des amines aliphatiques en C₈ à C₃₀, mono- ou polyinsaturées,
(3) des alcools en C₈ à C₃₀ mono- ou polyinsaturés, ainsi que leurs esters avec des acides monocarboxyliques en C₁ à C₄ saturés,
(4) des alcènes en C₁₆ à C₃₀ terminaux ou médians,
C) 0 à 24,45% en poids d'autres monomères copolymérisables et
D) 0,05 à 5% en poids d'un ou plusieurs composés comportant au moins deux radicaux oléfiniquement insaturés dans la molécule comme agents de réticulation,
à titre d'agents épaississants ou dispersifs.

2. Utilisation de copolymères suivant la revendication 1, en vue de la préparation desquels on a utilisé, à titre de composant A, de l'acide acrylique, de l'acide méthacrylique, ou de l'anhydride d'acide maléique.

3. Utilisation de copolymères suivant les revendications 1 et 2, en vue de la préparation desquels on a mis en oeuvre, à titre de composant B), un ou plusieurs composés à longues chaînes avec des doubles liaisons oléfiniques isolées, appartenant au groupe formé par
(1) des acides monocarboxyliques en C₁₄ à C₂₄, mono- à quadri insaturés, ainsi que leurs sels avec des métaux alcalins et des métaux alcalino-terreux, leurs esters alkyliques en C₁ à C₄, leurs esters avec la glycérine, ou leurs esters avec des polyglycérines,
(2) des amines en C₁₄ à C₂₄ primaires aliphatiques, mono- à quadri insaturées,
(3) des alcools en C₁₄ à C₂₄ primaires, mono- à quadri insaturés, ainsi que leurs esters avec des acides monocarboxyliques en C₁ à C₄ saturés,
(4) des alcènes en C₁₆ à C₂₄ terminaux.

4. Utilisation de copolymères suivant l'une quelconque des revendications 1 à 3, en vue de la préparation desquels on a utilisé, à titre de composant D, l'éther allylique de la pentaérythrite, le triméthylolpropane, ou le saccharose, avec au moins deux unités éther allylique dans la molécule, ainsi que le méthacrylate d'allyle, le (méth)acrylate d'oléyle, ou le méthylènebisacrylamide.

5. Utilisation de copolymères suivant la revendication 1, à titre d'agents épaississants ou d'agents dispersifs dans des préparations cosmétiques.

6. Utilisation de copolymères suivant la revendication 1, à titre d'agents épaississants ou d'agents dispersifs dans des préparations pharmaceutiques.

7. Copolymères que l'on peut obtenir par la polymérisation amorcée par voie radicalaire de
(A) 75 à 99,45% en poids d'un acide monocarboxylique en C₃ à C₅, oléfiniquement insaturé, d'un acide dicarboxylique en C₄ à C₈, oléfiniquement insaturé, ou de son anhydride, ou d'un mélange de tels acides carboxyliques ou anhydrides d'acides carboxyliques avec
(B) 0,5 à 24,95% en poids d'un ou plusieurs composés à longues chaînes avec de multiples liaisons C-C, appartenant au groupe formé par
(1) des acides monocarboxyliques en C₈ à C₃₀, mono- ou polyinsaturés, qui peuvent porter des radicaux hydroxyle complémentaires, ainsi que leurs sels de métaux alcalins et de métaux alcalino-terreux, leurs esters alkyliques en C₁ à C₄, leurs amides, leurs esters avec le sorbitan, leurs esters avec la glycérine, ou leurs esters avec des polyglycérines,
(2) des amines aliphatiques en C₈ à C₃₀, mono- ou polyinsaturées,
(3) des alcools en C₈ à C₃₀ mono- ou polyinsaturés, ainsi que leurs esters avec des acides monocarboxyliques en C₁ à C₄ saturés,
C) 0 à 24,45% en poids d'autres monomères copolymérisables et
D) 0,05 à 5% en poids d'un ou plusieurs composés comportant au moins deux radicaux oléfiniquement insaturés dans la molécule comme agents de réticulation, à titre d'agents épaississants ou dispersifs.

8. Préparations cosmétiques ou pharmaceutiques qui contiennent des copolymères suivant l'une quelconque des revendications 1 à 4, à titre d'agents épaississants et d'agents dispersifs en les proportions habituelles, à cette fin.
